Europäisches Patentamt

European Patent Office

Office européen des brevets

(11) Publication number: 0 153 522
A1

# EUROPEAN PATENT APPLICATION

(21) Application number: 84303973.6

(22) Date of filing: 13.06.84

(51) Int. Cl.⁴: C 07 C 55/02
C 07 C 55/21, C 07 C 51/245

(30) Priority: 29.06.83 US 509279

(43) Date of publication of application:
04.09.85 Bulletin 85/36

(84) Designated Contracting States:
BE DE FR GB IT NL

(71) Applicant: THE PROCTER & GAMBLE COMPANY
301 East Sixth Street
Cincinnati Ohio 45201(US)

(72) Inventor: O'Brien, David Allen
8741-A Harperspoint Drive
Cincinnati Ohio 45242(US)

(74) Representative: Brooks, Maxim Courtney et al,
Procter & Gamble (NTC) Limited Whitley Road
Longbenton
Newcastle-upon-Tyne NE12 9TS(GB)

(54) Process for the oxidation in the air of keto-acids in an organic diluent melt with a manganese catalyst.

(57) A process for oxidizing ketoacids in an organic diluent melt solution with air (molecular oxygen) in the presence of a manganese salt catalyst. The process provides 85% to 95% yields of selected diacids based on the reacted ketoacids. The preferred process provides high yields of dodecanedioic, undecanedioic and decanedioic diacids from 12-ketostearic acid (12-KSA) in a stearic acid diluent melt solution.

EP 0 153 522 A1

0153522

# AIR OXIDATION OF KETOACIDS IN ORGANIC DILUENT
## MELT WITH MANGANESE CATALYST

David A. O'Brien

## BACKGROUND OF THE INVENTION

The long-chain dicarboxylic acids, dodecanedioic, undecanedioic and decanedioic acids, and their derivatives have considerable utility. These diacids may be subsequently converted to diperoxyacids which are useful bleach compounds. In addition, some of the esters of these acids may be used as synthetic lubricants or as plasticizers. The acids produced by the present invention also provide valuable chemical intermediates for the manufacture of resins such as polyesters and the like.

Autoxidation and enolization of acetophenone in an acetic acid diluent with a Mn-catalyst is reported in a Dutch paper by H. J. Den Hertog, Jr., et al., published in the Journal of Catalysis, 6, pp. 357-361 (1966). Alkyl chain length selectivity is not a problem in the oxidation of acetophenone because it lacks an alkyl chain of more than one carbon adjacent to the ketone carbonyl. The rate of this oxidation is also enhanced by the presence of the benzene ring adjacent to the carbonyl group.

Specific autoxidations of ketones using manganese stearate as a catalyst are reported in a Russian paper by I. I. Korsak et al., Ser. Khim. Navuk, 1979, (1), pp. 37-40. Oxidation of ketoacids under conditions set out in this Russian paper resulted in selected yields of diacids of less than 50%.

U.S. Pat. No. 2,847,432 to T. R. Steadman and J. O. H. Peterson, issued August 12, 1958, describes a process for oxidizing an alkyl ester of 12-ketostearic acid which comprises reacting said compound with a nitric acid solution in the presence of a mixed catalyst containing a copper-bearing material and a compound of vanadium. The reaction mixture contains dodecanedioic and undecanedioic acids. Difficulties are associated with nitric acid oxidation processes. Nitric acid is corrosive, produces a toxic waste stream upon reaction, and, in net, is expensive to use.

0153522

- 2 -

## SUMMARY OF THE INVENTION

A process for oxidizing ketoacids in an organic diluent melt solution with air bubbles (molecular oxygen) in the presence of a manganese salt catalyst. The process provides selected diacids. The preferred process provides high yields of dodecanedioic, undecanedioic and decanedioic diacids from a solution of 12-ketostearic acid (12-KSA) and stearic acid diluent.

## OBJECTS

A principal object of the present invention is to provide a process for producing dicarboxylic acids from ketoacids.

Another object of the present invention is to provide a process for producing dicarboxylic acids from esters of ricinoleic acid, such as castor oil.

Another object of the invention is to provide a process for producing dicarboxylic acids from 12-ketostearic acid.

Still another object of the invention is to provide a process for producing acid mixtures comprised essentially of dodecanedioic, undecanedioic, and decanedioic acids from 12-ketostearic acid.

Other objects of the invention will in part be obvious and will in part appear hereinafter.

## DETAILED DESCRIPTION OF THE INVENTION

The invention accordingly comprises the process involving the several steps and the relation and the order of one or more of such steps with respect to each of the others which are exemplified in the following detailed disclosure, and the scope of the application of which will be indicated in the claims.

For a fuller understanding of the nature and objects of the invention, reference should be had to the following detailed description.

There are four essential ingredients in the practice of the present invention: ketoacids, organic diluent, manganese catalyst, and molecular oxygen.

The ketoacids are $C_8$ to $C_{22}$ alkyl ketoacids. The preferred ketoacids are $C_{16}$ to $C_{20}$ alkyl ketoacids which provide selected $C_3$ to $C_{18}$ diacids. 12-ketostearic acid is the most preferred. It can be obtained from castor oil via a process set out in U.S. Pat. No. 2,847,432 to T. R. Steadman and J. O. H. Peterson, issued August 12, 1958. The ketoacids are melted and mixed with the catalyst and the organic diluent.

The manganese catalyst is a $C_{12}$ to $C_{22}$ alkyl fatty acid salt. It is used at a level of about 0.02% to about 1% by weight of soluble manganese ion in the reaction solution. A level of 0.05% to 0.2% is preferred. Manganese stearate is the preferred catalyst.

The organic diluent is preferably selected from $C_8$ to $C_{22}$ fatty acids, and $C_{12}$ to $C_{30}$ hydrocarbons. More preferably the diluent is $C_{12}$ to $C_{22}$ fatty acids which are primarily saturated. Other less preferred organic diluents, such as acetic acid, can be used. The key is that it should be miscible with the ketoacid, not participate in or promote undesired side reactions, and should itself withstand the reaction conditions.

The oxygen source is preferably air which is bubbled through the reaction solution at a flow rate selected for efficient oxidation. Pure oxygen or a gas containing it can be used, but air is cheaper and preferred.

The reaction is carried out at 85°C to 140°C, preferably 95°C to 110°C. For the preferred 12-ketostearic acid (12-KSA) oxidation is done by using air bubbles, manganese stearate, and stearic acid with the ratio of 12-KSA to stearic acid being from 1:9 to 4:1, preferably from 2.5:1 to 1:2.5. The preferred temperature is 100°C.

A source of 12-ketostearic acid (12-KSA) is castor oil. Castor oil, a plentiful and cheap commodity, is comprised essentially of ricinolein, i.e., the triglyceride of ricinoleic acid. Ricinoleic acid or esters thereof contain both a hydroxy group and an unsaturated group. By the present invention, it is possible to obtain from esters of ricinoleic acid the valuable dicarboxylic acids containing 10, 11 and 12 carbon atoms and the valuable monocarboxylic acids containing 5, 6 and 7 carbon atoms.

Thus, castor oil or a ricinoleate is isomerized to produce a 12-ketostearate which is then hydrolyzed to produce 12-ketostearic acid which is subsequently oxidatively cleaved to a mixture of mono- and dicarboxylic acids. The isomerization of a ricinoleate is preferably carried out in the presence of an inert support carrying a catalyst selected from the group consisting of palladium, platinum, rhodium and ruthenium and at a temperature between 200°C and 300°C. The reaction is also preferably carried out in a closed system. See U.S. Pat. No. 2,847,432, Steadman et al., issued August 12, 1958, for more details.

The 12-ketostearic acid formed by the above isomerization and hydrolysis reactions is then subjected to an oxidative cleavage to produce a mixture comprised essentially of mono- and dicarboxylic acids. This acid mixture comprises a dicarboxylic acid, dodecanedioic acid, which contains the same number of carbon atoms between the carboxyl groups as the number of carbon atoms between the keto group and the carboxyl group of the 12-ketostearic acid starting material, also a dicarboxylic acid, undecanedioic acid, which contains one less carbon atom than the first-mentioned dicarboxylic acid, and lesser amounts of decanedioic acid which contains 2 less carbon atoms than the first-mentioned dicarboxylic acid. There are also present the corresponding lower monocarboxylic acids, caproic and enanthic acids, from the other portion of the molecule being oxidized. These volatile monobasic acids are very useful in the manufacture of plasticizers and synthetic lubricants.

The production of the desired dicarboxylic acids is preferably achieved by cleaving 12-ketostearic acid with oxygen at a temperature between about 85°C and 140°C in the presence of a soluble manganese catalyst in an organic diluent. In one preferred embodiment of the invention, the 12-ketostearic acid obtained from the isomerization of castor oil and the dicarboxylic acids produced therefrom are dodecanedioic, undecanedioic and decanedioic acids. This preferred embodiment of the invention calls for the cleavage reaction to be carried out in the presence of manganese at a level of about 0.02% to about 1% of soluble manganese by weight of the charge, i.e., the reaction solution.

## EXAMPLES

Specific detailed methods for practicing the present invention are set forth in the following nonlimiting examples.

## EXAMPLE I

A bubble reactor was constructed from a glass cylinder of approximately 150 mm in length and 24 mm in diameter. The bottom of this cylinder was fitted with a 20 mm diameter glass sparge tube with 10-20 m size pores. Into this reactor were weighed 3.50 gms of 12-ketostearic acid and 3.50 gms of stearic acid diluent. The 12-ketostearic acid was obtained by the isomerization of castor oil with a palladium catalyst as taught by the cited Steadman et al. patent followed by the hydrolysis of the resulting triglyceride, and the isolation of the 12-ketostearic acid by recrystallization from methanol. The stearic acid diluent used was reagent grade, fully saturated, and contained 2% palmitic acid.

The reactor containing the stearic and 12-ketostearic acids was placed in a 100°C oil bath. Air was bubbled through the reactor at a rate of 215 mls/minute as measured under ambient conditions going into the reactor. Next, 0.088 gm of manganese (II) stearate catalyst was added to the reactor, and an exit gas line leading to traps for evolved water and carbon dioxide was attached to the top of the reactor. The manganese (II) ion in the melt was about 0.11%. Aliquots of approximately 0.25 gm were taken from the reactor at the intervals listed below, silated, and analyzed by gas-liquid chromatography. Lauric acid was used as an internal standard.

It will be noted in Table 1 that at about 60 minutes the best selectivity was found. Of the $C_{10}-C_{12}$ diacids found in the 60 minute sample, 41% was $C_{12}$ diacid, 43% was $C_{11}$ diacid, and 16% was $C_{10}$ diacid. This distribution was about the same for the other samples in Table 1.

## TABLE 1

| Time (Min.) | Corrected Weight Fractions[1] | | Selectivity[2] for $C_{10}$-$C_{12}$ Diacids |
|---|---|---|---|
| | Ketoacid Consumed | $C_{10}$-$C_{12}$ Diacids | |
| 30 | 26.1% | 12.0% | 71.5% |
| 60 | 38.4 | 23.4 | 93.9 |
| 120 | 66.8 | 33.5 | 78.0 |
| 180 | 80.2 | 40.1 | 77.9 |
| 240 | 87.9 | 42.6 | 75.4 |
| 300 | 92.8 | 43.5 | 74.2 |

[1]Grams of $C_{10}$-$C_{12}$ diacids produced per gram of reactant (12-ketostearic acid) originally charged to the reactor. The weights of the diluent and catalyst are excluded from the total sample weight upon which the percentage of diacids is calculated. Included in the total sample weight are the short (mostly $C_5$-$C_7$) monoacids which are relatively volatile and partially stripped from the reactor by the air stream. This lost weight was calculated from the fact that 1 mole of monoacid is produced for each mole of diacid produced by oxidative cleavage of a ketoacid.

[2]Selectivity is moles of $C_{10}$-$C_{12}$ diacids produced per mole of 12-ketostearic acid consumed.

## EXAMPLE II

The same equipment, materials, and conditions described in Example I were employed, except that the temperature was set at 120°C and a charge of 7.0 gms of 12-ketostearic acid was substituted for the 50:50 mixture of stearic acid and 12-ketostearic acid. Also, the level of manganese stearate catalyst was slightly higher (0.14 gm). These conditions corresponded essentially to the preferred conditions taught by Korsak et al. for the oxidation of simple ketones. The acid functionality taught by Korsak et al. is supplied by the ketoacid itself. As in Example I aliquots were taken over time and analyzed by g.l.c.

### TABLE 2

| Time (Min.) | Corrected Weight Fractions[1] | | Selectivity[2] |
| | Ketoacid Consumed | $C_{10}$-$C_{12}$ Diacids | for $C_{10}$-$C_{12}$ Diacids |
| --- | --- | --- | --- |
| 30 | 11.2% | 2.5% | 34.6% |
| 60 | 54.8 | 14.2 | 40.3 |
| 90 | 79.9 | 23.7 | 44.8 |
| 120 | 93.1 | 28.1 | 47.0 |
| 180 | 99.9 | 30.1 | 46.8 |

[1]Grams of $C_{10}$-$C_{12}$ diacids produced per gram of reactant (12-ketostearic acid) originally charged to the reactor. The weights of the diluent and catalyst are excluded from the total sample weight upon which the percentage of diacids is calculated. Included in the total sample weight are the short (mostly $C_5$-$C_7$) monoacids which are relatively volatile and partially stripped from the reactor by the air stream. This lost weight was calculated from the fact that 1 mole of monoacid is produced for each mole of diacid produced by oxidative cleavage of a ketoacid.

[2]Selectivity is moles of $C_{10}$-$C_{12}$ diacids produced per mole of 12-ketostearic acid consumed.

As shown in Example II, the oxidation of 12-KSA under the Korsak et al. conditions yielded less than 50% selected $C_{10}$-$C_{12}$ diacids. This assumes that the ketoacid provides the acid moiety provided by the stearic acid catalyst taught by Korsak et al. It was surprising that 85% to 95% selectivity could be obtained under the conditions of this invention, as illustrated in Example I.

CLAIMS

1. A process for oxidizing a $C_8$-$C_{22}$ alkyl ketoacid characterized by reacting the ketoacid with free molecular oxygen in the presence of a soluble manganese salt catalyst in an organic diluent melt solution, at a temperature in the range from 85°C to 140°C, wherein the ketoacid and diluent have a weight ratio of from 1:9 to 4:1; and wherein the manganese catalyst is present in the melt at a level of from 0.02 to 1 wt. % soluble manganese ion.

2. A process according to Claim 1 wherein the catalyst is manganese stearate and the diluent is stearic acid.

3. A process for making a diacid or mixture of diacids by oxidation of a ketoacid comprising the steps of:
   A. preparing a homogeneous melt solution of:
      1. $C_8$-$C_{22}$ alkyl ketoacid,
      2. an organic carrier diluent selected from $C_8$-$C_{22}$ fatty acids and $C_{12}$-$C_{30}$ saturated hydrocarbons, and
      3. a soluble manganese salt catalyst;
   wherein the ketoacid and diluent have a weight ratio of from 1:9 to 4:1; and wherein the manganese catalyst is present in the melt at a level of from 0.02 to 1 wt. % soluble manganese ion;
   B. oxidizing the ketoacid with a gas containing free oxygen at a temperature in the range of 85° to 140°C;
wherein the oxidation provides a yield of from 85% to 95% of diacid.

4. A process according to any of Claims 1 to 3 wherein the ketoacid has a carbon chain length of 16 to 20 carbon atoms and the diacid or diacids made thereby have a carbon chain length of 3 to 18 carbon atoms.

5.  A process according to any of Claims 1 to 4 wherein the organic diluent carrier is stearic acid and the ketoacid to diluent ratio is from 2.5:1 to 1:2.5.

6.  A process according to any of Claims 1 to 5 wherein the manganese catalyst is a manganese salt of a $C_2-C_{22}$ carboxylic acid.

7.  A process according to any of Claims 1 to 6 wherein the ketoacid is 12-ketostearic acid.

8.  A process according to any of Claims 1 to 7 wherein the temperature is in the range from 95°C to 110°C.

9.  A process for preparing a mixture of dicarboxylic acids from a melt solution of 12-ketostearic acid and stearic acid diluent which comprises heating the melt to a temperature of from 85°C to 140°C and reacting the 12-ketostearic acid with molecular oxygen ($O_2$) which is bubbled through the melt solution in the presence of a soluble manganese salt catalyst, the catalyst being present at a level of from 0.02% to 1%, preferably 0.05% to 0.2% manganese ion by weight of the solution, and recovering from the reaction mixture a mixture containing primarily dodecanedioic, undecanedioic and decanedioic diacids.

European Patent Office

**EUROPEAN SEARCH REPORT**

. Application number

EP 84 30 3973

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-3 833 625  (BERTRAND et al.) <br> * Claims 1-8; column 2, lines 9-36, 40-55; column 3, lines 18-30; column 6, example 12 * | 1,3,8 | C 07 C 55/02 <br> C 07 C 55/21 <br> C 07 C 51/245 |
| | --- | | |
| D,Y | VESTSI AKAD. NAVUK BSSR; SER. KHIM. NAVUK, 1979, (1), pages 37-40; I.I. KORSAK et al.: "Liquid-phase catalytic oxidation of aliphatic ketones" * Whole document * | 1,2,6, 8 | |
| | --- | | |
| A | GB-A-1 012 237  (SHELL) <br> * Claims 1,6,14,17-21 * | 1 | |
| | ----- | | |

TECHNICAL FIELDS SEARCHED (Int. Cl.4)

C 07 C 55/00
C 07 C 51/00

The present search report has been drawn up for all claims

| Place of search | Date of completion of the search | Examiner |
|---|---|---|
| THE HAGUE | 21-12-1984 | KLAG M. J. |